# EUROPEAN PATENT APPLICATION

(11) **EP 2 332 442 A1**
(43) Date of publication of application: **15.06.2011**
(21) Application number: 09816323.1
(22) Date of filing: 16.04.2009
(51) Int. Cl.: A45D 40/26

(54) **COSMETIC CONTAINER WITH IONTOPHORESIS DEVICE**

(30) Priority: 24.09.2008 KR 20080093653
(71) Applicant: Yonwoo Co., Ltd, Incheon 404-250 (KR)
(72) Inventor: KI, Joong-Hyun, Incheon 404-250 (KR)
(74) Representative: Patentanwaltskanzlei WILHELM & BECK
(86) International application number: PCT/KR2009/001986
(87) International publication number: WO 2010/035930

(57) **Abstract**

The cosmetic container with an ion introduction unit is disclosed, which cosmetic container comprises a streamline shaped lower casing which has a button engaging hole formed at one side and passing through; a cosmetic storing container which includes a housing which is installed in the interior of the lower casing and stores cosmetics therein, with a piston being installed at a lower side of the same; a fixture which is inserted into an upper side of the housing and has an insertion hole passing through the center of the same; a piston rod, of which center portion is vertically passed through, with a lower end of the same being inserted into the insertion hole, with an upper portion of the same having a diameter larger than the insertion hole; a recovery spring which is installed between the fixture and the upper side of the piston rod; and a dispenser which has a diameter larger than the insertion hole and is inserted into a lower side of the piston rod and has a check valve at its lower side while being opened and closed by means of a pressure of cosmetics; a button which is installed at a lower side of the fixture and of which one side is inserted into the button engaging hole; an upper casing which is installed at an upper side of the lower casing and has a passing-through cosmetic discharge port connected with an upper side of the piston rod for thereby discharging cosmetics; and an ion introduction unit which is installed at an upper side of the upper casing and penetrates cosmetics into a skin with the aid of potential difference, with a power switch being installed at one side of the lower casing.

## Description

### Technical Field

The present invention relates to a cosmetic container with an ion introduction unit, and in particular to a cosmetic container with an ion introduction unit in which a cosmetic casing and an ion introduction unit are formed in a singular structure and cosmetics can be applied on a skin together with ions.

A lower casing is made in a streamline shape, which results in an easier holding of the cosmetic container.

### Background Art

A person uses an ion introduction unit in order to apply a cosmetic or a skin supplement product into a deeper portion of a skin when the user applies a certain cosmetic, a skin supplement product or something in order to effectively care a skin, in particular, a face skin. The ion introduction unit emits small electric currents which helps apply a cosmetic product or a skin supplement product into a deeper portion of a skin with the aid of an electric repulsive force of electric charges. The ion introduction unit is widely used for its excellent effects.

However, the conventional ion introduction unit has a problem that cosmetics do not well discharge. So, the user of the conventional ion introduction unit has to directly apply a cosmetics or a skin supplement product with a hand, and the user uses the ion introduction unit separately from the use of cosmetics. The user is needed to remove part of the cosmetics applied on the hand when using the ion introduction unit, which causes a lot of inconveniences when in use and results in an increase of time needed for makeup.

Since the conventional ion introduction unit is simply made in a cylindrical shape at a portion where a user usually holds, a user feels uneasy when he holds the ion introduction unit with hands.

### Disclosure of Invention

Accordingly, it is an object of the present invention to overcome the above problems and to provide a cosmetic container with an ion introduction unit in which a cosmetic casing and an ion introduction unit are formed in a singular structure and cosmetics can be directly applied on a skin together with ions.

In the present invention, a lower casing is made in a streamline shape, which results in an easier holding of the cosmetic container.

To achieve the above objects, there is provided a cosmetic container with an ion introduction unit which comprises a streamline shaped lower casing which has a button engaging hole formed at one side and passing through; a cosmetic storing container which includes a housing which is installed in the interior of the lower casing and stores cosmetics therein, with a piston being installed at a lower side of the same; a fixture which is inserted into an upper side of the housing and has an insertion hole passing through the center of the same; a piston rod, of which center portion is vertically passed through, with a lower end of the same being inserted into the insertion hole, with an upper portion of the same having a diameter larger than the insertion hole; a recovery spring which is installed between the fixture and the upper side of the piston rod; and a dispenser which has a diameter larger than the insertion hole and is inserted into a lower side of the piston rod and has a check valve at its lower side while being opened and closed by means of a pressure of cosmetics; a button which is installed at a lower side of the fixture and of which one side is inserted into the button engaging hole; an upper casing which is installed at an upper side of the lower casing and has a passing-through cosmetic discharge port connected with an upper side of the piston rod for thereby discharging cosmetics; and an ion introduction unit which is installed at an upper side of the upper casing and penetrates cosmetics into a skin with the aid of potential difference, with a power switch being installed at one side of the lower casing.

The lower casing comprises a slippery prevention part made of a rough material and formed at one side of the same and having protrusions on its surface.

The ion introduction unit comprises a power supply battery which is installed in the interior of the lower casing and supplies power to the ion introduction part; and a wireless charger which is installed in the interior of the lower casing and charges the power supply battery by receiving external electric power.

The ion introduction unit comprises a Light Emitting Diode (LED) display part which is installed at one side of the lower casing and displays the charging state of the ion introduction unit.

### Advantageous Effects

According to the present invention, a cosmetic casing and an ion introduction unit are formed in a singular structure and cosmetics can be directly applied on a skin advantageously together with ions.

A lower casing is made in a streamline shape, which results in an easier holding of the cosmetic container.

### Brief Description of the Drawings

The present invention will become better understood with reference to the accompanying drawings which are given only by way of illustration and thus are not limitative of the present invention, wherein;

Figure 1 is a perspective view illustrating a cosmetic container with an ion introduction unit according to a preferred embodiment of the present invention;

Figure 2 is a front view illustrating a cosmetic container with an ion introduction unit according to a preferred embodiment of the present invention;

Figure 3 is a side cross sectional view illustrating a cosmetic container with an ion introduction unit according to a preferred embodiment of the present invention; and

Figure 4 and 5 are a view of an operation state of a cosmetic container with an ion introduction unit according to a preferred embodiment of the present invention.

### Best modes for carrying out the invention

The preferred embodiments of the present invention will be described with reference to the accompanying drawings. The same elements shown in each drawing will be given the same reference numerals.

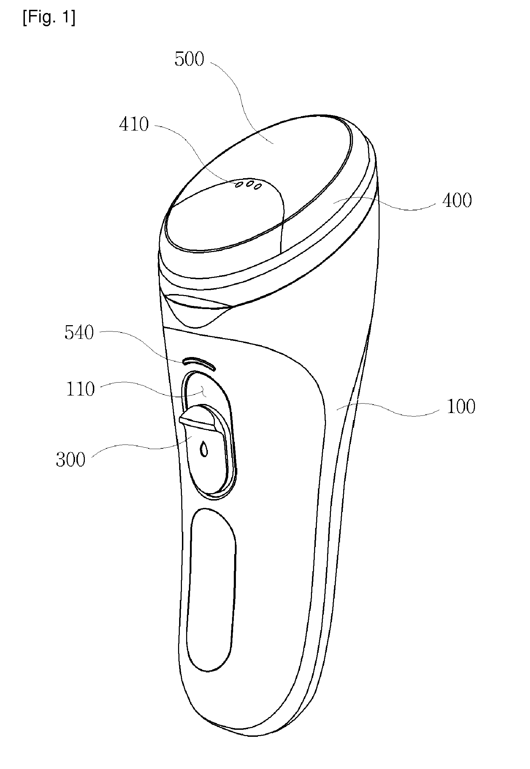

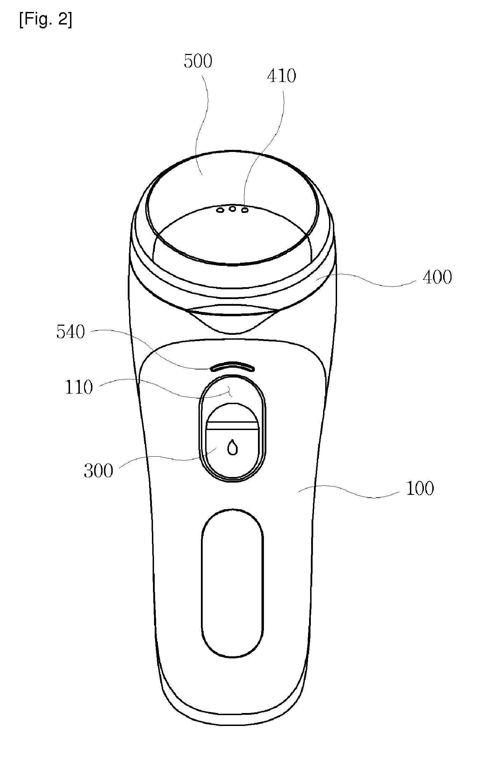

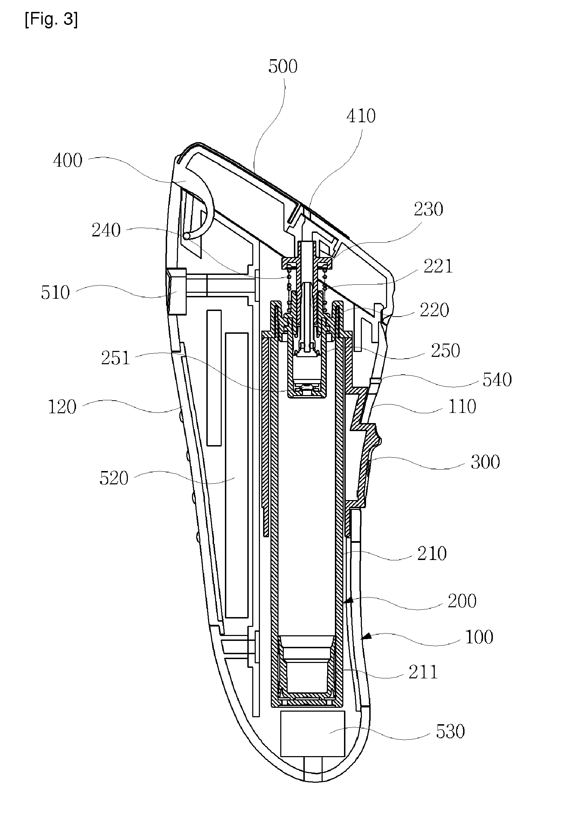

Figure 1 is a perspective view illustrating a cosmetic container with an ion introduction unit according to a preferred embodiment of the present invention, and Figure 2 is a front view illustrating a cosmetic container with an ion introduction unit according to a preferred embodiment of the present invention, and Figure 3 is a side cross sectional view illustrating a cosmetic container with an ion introduction unit according to a preferred embodiment of the present invention.

As shown in Figures 1 to 3, the cosmetic container with an ion introduction unit according to a preferred embodiment of the present invention comprises a lower casing 100, a cosmetic storing container 200, a button 300, an upper casing 400, and an ion introduction unit 500. There are further provided a slippery prevention part 120, a power supply battery 520, a wireless charger 530, and a LED display unit 540.

The lower casing 100 stores cosmetics and is formed in a streamline shape for an easier holding by a user. A button engaging hole 110 passes through one side of the lower casing 100. Since a button 300, which will be described later, is engaged to the button engaging hole 110, the user can use cosmetics by pressing upwards the button 300 with a thumb while holding the lower casing 100 with a hand. At this time, the button engaging hole 110 passes through at a certain height at one side of the lower casing 100, which facilities the button 300 to move vertically.

A slippery prevention part 120 has protrusions and is installed at one side surface of the lower casing 100. When the user holds the lower casing 100, the lower casing 100 does not well escape from the user's hand with the aid of the same. It is preferred that the slippery prevention part 120 is installed at the other side surface of the button engaging hole 110 to be placed at the finger portions except for the thumb when the user holds the lower casing 100 and is made of tough materials so as to have a lot of friction force.

The cosmetic storing container 200 is installed in the interior of the lower casing 100 and stores cosmetics therein. When the button 300 is pressed upwards, cosmetics are discharged. The cosmetic storing container 200 is assembled or disassembled from the lower casing 100. When the cosmetics run out, it can be replaced with a new container for thereby refilling the cosmetics. The cosmetic storing container 200 comprises a housing 210, a fixture 220, a piston rod 230, a recovery spring 240 and a dispenser 250.

The housing 210 is shaped in a container shape to store cosmetics therein. The cosmetic storing container 200 stores various cosmetics (eye cream, canceller, lip gloss, etc.). The cosmetic storing container 200 is generally made of a synthetic resin of plastic, with a piston 211 being installed at a lower side of the same. Since the piston 211 is contracted by as much height as the volume of the used cosmetics as the user uses cosmetics, the piston 2100 protruded beyond the top of the housing 210. The cosmetics remain always on the top of the housing 210 until the cosmetics run out. The piston 211 is made of flexible material, and the portion where the cosmetics are stored is air-tightly separated in the interior of the housing 210, so it can be possible for the portion where cosmetics are stored, to be in a vacuum state.

The fixture 220 is fixed to the upper side of the housing 210, with an insertion hole 221 being formed at the center portion in a vertical direction. The fixture 220 seals the cosmetics stored in the interior of the housing 210, and the piston rod 230, which will be described later, is inserted into the insertion hole 221.

The piston rod 230 is inserted into the insertion hole 221, and the diameter of the center portion of the piston rod 230 is the same as the diameter of the insertion hole 221 so that the cosmetics stored in the housing 210 do not discharge to the outside. The diameter of the upper side of the piston rod 230 is larger than the diameter of the insertion hole 221, so that the piston rod 230 is prevented from inserting into the insertion hole 221 and escaping into the interior of the housing 210.

The recovery spring 240 is installed between the fixture 220 and the piston rod 230. The recovery spring 240 is to recover the fixture 220 to its original position with the aid of an elastic recovery force of the spring after the fixture 220 is pressed upwards by means of the button 230 which will be described later.

The dispenser 250 has a diameter larger than the insertion hole 221 and is inserted into a lower side of the piston rod 230. The dispenser 250 prevents the piston rod 230 from escaping toward the upper side of the insertion hole 221. A check valve 251 is installed at a lower side of the dispenser 250. When pressure is applied to the check valve 251, the cosmetics are inputted into the interior of the dispenser 250. The inputted cosmetics are discharged to the outside via the cosmetic discharge port 410 through the center portion of the piston rod 230.

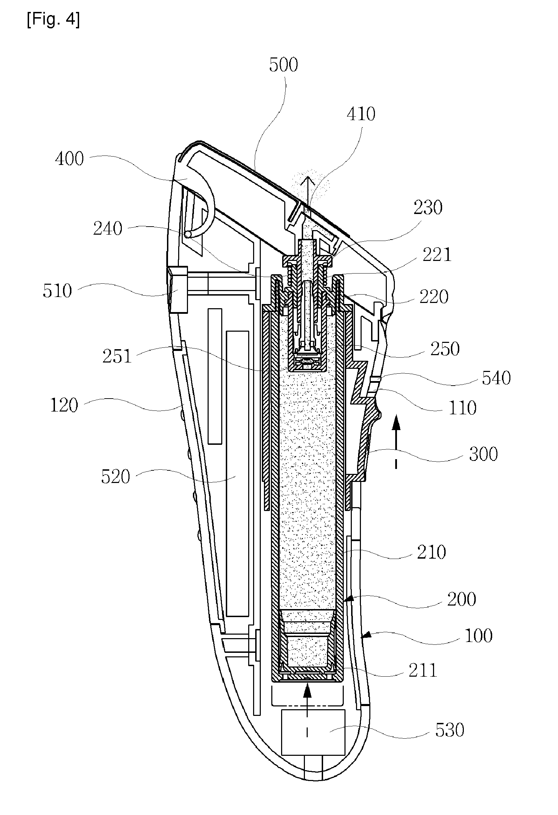

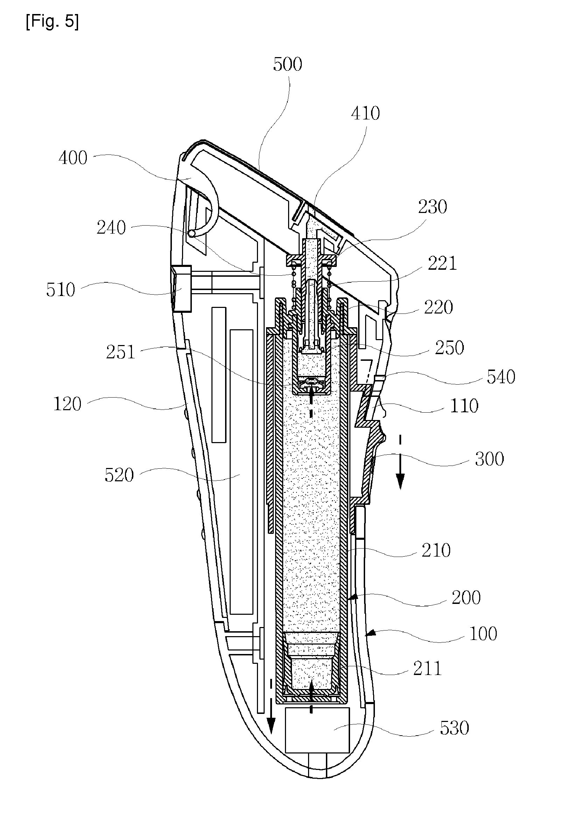

The button 300 is installed at a lower side of the fixture 220, with one side of the same being installed at the button engaging hole 110. The button 300 surrounds the outer sides of the housing 210 and is installed at a lower side of the fixture 220. When the user pressures the button 300 upwards, the housing 210 and the fixture 220 concurrently move upward. As shown in Figure 4, since the upper side of the piston rod 230 is fixed by means of the upper casing 400, the inner space of the dispenser 250 is compressed, which results in discharging cosmetics. As shown in Figure 5, the button 300, the housing 210 and the fixture 220 move downwards by means of an elastic recovery force of the recovery spring 240. At this time, since the inner space of the dispenser 250 increases, the check valve 250 opens by the pressure of the cosmetics, and the cosmetics fill into the inner space of the dispenser 250.

The upper casing 400 is installed at an upper side of the lower casing 100, and the cosmetics are discharged via the cosmetic discharge port 410. The cosmetic discharge port 410 passes through the upper side of the piston rod 230 and receives the cosmetics from the upper side of the piston rod 230 and discharges the same to the outside.

The upper casing 400 is installed at an upper side of the lower casing 100 in an opening and closing structure. When the cosmetics stored in the cosmetic storing container 200 runs out by a user, the user opens the upper casing 400 and easily exchanges the cosmetic storing container 200 with new one.

The ion introduction unit 500 is installed at an upper side of the upper casing 400 for performing an ion introduction function by emitting small electric currents. The ion introduction part 500 operates by receiving power from the power switch 510 installed at one side of the lower casing 100. Since the ion introduction unit 500 emits small electric currents, electric repulsive force is generated from the electric charges of the cosmetics and the skin supplement products, which helps penetrate the cosmetics or skin supplement products into the deeper portions of a user's skin. The ion introduction unit 500 is easy to use since the user presses the button 300 upwards and instantly uses the cosmetics from the cosmetic discharge port 410, while maximizing the effects that the cosmetics penetrate into the user's skin, which are the major features of the present invention.

The ion introduction unit 500 might be configured to be electrically charged in a wireless method by installing a power supply battery 520 and a wireless charger 530 at one side of the lower casing 100 for thereby supplying electric power to the ion introduction unit 500. When introducing ions from the ion introduction unit 500, it is preferred that an external power adaptor (not shown) is connected to one side of the ion introduction unit 500. The power supply battery 520 and the wireless charger 530 are installed in the interior of the lower casing 100, so the user can easily use the ion introduction unit 500 wirelessly without installing the external power adaptor (not shown). The wireless charger 530 receives electric power from the outside and charges external power to the power supply battery 520, so the user can easily use the ion introduction unit 500 anywhere.

A LED display part 540 is installed at one side of the lower casing 100 for thereby displaying the charging state of the ion introduction unit 500. The LED display part 540 is connected to one side of the power supply battery 520 for thereby receiving electric power from the same. It is preferred that the LED display part 540 is designed to change its emission color depending on the charging level of the same, so that the user can easily recognize the timing of charge.

As the present invention may be embodied in several forms without departing from the spirit or essential characteristics thereof, it should also be understood that the above-described examples are not limited by any of the details of the foregoing description, unless otherwise specified, but rather should be construed broadly within its spirit and scope as defined in the appended claims, and therefore all changes and modifications that fall within the meets and bounds of the claims, or equivalences of such meets and bounds are therefore intended to be embraced by the appended claims.

## Claims

1. A cosmetic container with an ion introduction unit, comprising:
a streamline shaped lower casing which has a button engaging hole formed at one side and passing through;
a cosmetic storing container which includes:
a housing which is installed in the interior of the lower casing and stores cosmetics therein, with a piston being installed at a lower side of the same;
a fixture which is inserted into an upper side of the housing and has an insertion hole passing through the center of the same;
a piston rod, of which center portion is vertically passed through, with a lower end of the same being inserted into the insertion hole, with an upper portion of the same having a diameter larger than the insertion hole;
a recovery spring which is installed between the fixture and the upper side of the piston rod; and
a dispenser which has a diameter larger than the insertion hole and is inserted into a lower side of the piston rod and has a check valve at its lower side while being opened and closed by means of a pressure of cosmetics;
a button which is installed at a lower side of the fixture and of which one side is inserted into the button engaging hole;
an upper casing which is installed at an upper side of the lower casing and has a passing-through cosmetic discharge port connected with an upper side of the piston rod for thereby discharging cosmetics; and
an ion introduction unit which is installed at an upper side of the upper casing and penetrates cosmetics into a skin with the aid of potential difference, with a power switch being installed at one side of the lower casing.

2. The container according to claim 1, wherein said lower casing comprises a slippery prevention part formed at one side of the same and having protrusions on its surface.

3. The container according to claim 1, wherein said ion introduction unit comprises:
a power supply battery which is installed in the interior of the lower casing and supplies power to the ion introduction part; and
a wireless charger which is installed in the interior of the lower casing and charges the power supply battery by receiving external electric power.

4. The container according to claim 1, wherein said ion introduction unit comprises a Light Emitting Diode (LED) display part which is installed at one side of the lower casing and displays the charging state of the ion introduction unit.
